# EUROPEAN PATENT SPECIFICATION

(11) **EP 4 486 585 B1**
(45) Date of publication and mention of the grant of the patent: **11.02.2026**
(21) Application number: 23719039.2
(22) Date of filing: 03.03.2023
(51) Int. Cl.: B60H 3/06, B60H 1/32, A23B 7/152, B01D 46/00

(54) **SYSTEM FOR PURIFYING AIR IN A CLOSED STORAGE/TRANSPORT SPACE**
SYSTEM ZUR REINIGUNG VON LUFT IN EINEM GESCHLOSSENEN SPEICHER-/TRANSPORTRAUM
SYSTÈME DE PURIFICATION D'AIR DANS UN ESPACE DE STOCKAGE/TRANSPORT FERMÉ

(30) Priority: 04.03.2022 FI 20225196
(43) Date of publication of application: 08.01.2025
(73) Proprietor: LUMIPROLINE FINLAND OY, 40400 Jyväskylä (FI)
(72) Inventor: LUHANKA, Karo, 40400 Jyväskylä (FI)
(74) Representative: Kespat Oy
(86) International application number: PCT/FI2023/050119
(87) International publication number: WO 2023/166249

(56) References cited:
- EP-A1- 3 847 894
- CN-A- 101 401 947
- KR-A- 20200 062 496
- KR-Y1- 200 403 240
- US-A1- 2007 041 882

## Description

The invention relates to a system for purifying air in a closed storage/transport space according to claim 1, which system comprises an air purifier comprising
- a casing with an air inlet opening and an air outlet opening, wherein an air flow channel lies between the air inlet opening and the air outlet opening inside the casing,
- a fan configured to suck air from the storage/transport space via the air inlet opening into the flow channel inside the air purifier and to blow purified air via the air outlet opening back into the storage/transport space,
- a filter system arranged in the flow channel in such a manner that air flows through the filter system, wherein the filter system comprises, as chemical filter material, both a UV-activated photocatalyst and a chemical filter material comprising potassium permanganate, and
- a UV-emitting light source for activating the photocatalyst of the filter system.

The transport of fresh fruit, vegetables, berries, flowers and other plants from the regions of production to the points of sale, by land and by sea, can take several days or even several weeks, during which time the plants are in a closed, refrigerated space. Moreover, plants can be stored in stores and wholesale warehouses for long periods of time before being sold. As part of their natural vital functions, plants produce ethylene (ethene, C₂H₄), which causes, *inter alia,* a rapid ripening of fruit, senescence in leaves and a wilting of flowers in plants, as well as changes in the taste of vegetables. In particular green plants are very sensitive to the adverse effects of ethylene, such as turning yellow. Ethylene is generated in significant amounts especially in ripening fruit and in ageing, corrupted and mouldy tissue. For example, bananas, apples, kiwis and tomatoes produce significant amounts of ethylene.

In addition to ethylene, air of a transport/storage space can also contain harmful bacteria such as Salmonella, Listeria and E. coli, Bacillus cereus, as well as viruses and moulds, which should be removed from the transport/storage space.

The transport and storage of plants in a closed place leads to an accumulation of ethylene in the space in which the plants are kept, the concentration of which can become quite high. A high ethylene concentration in a closed storage or transport space can lead to considerable spoilage.

The use of air purifiers in storage spaces in order to keep the ethylene concentration low and to remove other harmful substances is known. One suitable air-purifying device for the elimination of ethylene is known from patent publication US 2013/0202494 A1, in which an air-purifying device based on photocatalytic oxidation is disclosed.

When ultra-violet radiation with a wavelength of less than 400 nm is directed at a surface of a selected semiconductor, for example titanium dioxide (TiO₂) or zinc oxide (ZnO), the energy of the photons excites the electrons from the valence band to the conduction band. As a consequence, the short carbon chain of the ethylene breaks up very readily in a spontaneous chemical reaction on or very close to the surface of the semiconductor. The end products of the reaction are carbon dioxide and water.

However, a photocatalyst alone is not particularly effective for removing ethylene. In addition to a photocatalyst, it is known to use potassium permanganate to eliminate ethylene. The patent publication US 2014342064 A1 discloses the use of potassium permanganate and titanium dioxide for the removal of ethylene in a vegetable compartment of a refrigerator. The combination of a photocatalyst and potassium permanganate purifies air very effectively in particular in spaces for storing foodstuffs, as air can be effectively rid of viruses, bacteria and mould by means of a photocatalyst, especially titanium dioxide, while potassium permanganate is effective in removing ethylene.

A problem with known systems for purifying air in a closed storage/transport space is the handling and dimensioning of the filter material to meet the needs of different storage/transport spaces.

Further prior art can be found in documents US 2007/041882 A1 and EP 3 847 894 A1.

The object of the invention is to provide an improved system for purifying air in a storage/transport space of ethylene and other impurities, which reduces plant spoilage in the space in question. The characteristic features of this invention are indicated in the attached patent claim 1.

A system according to the invention for purifying air in a closed storage/transport space according to claim 1 comprises an air purifier, the air purifier including a casing with an air inlet opening and an outlet air opening, wherein an air flow channel lies between the air inlet opening and the air outlet opening inside the casing, a fan configured to suck air from the storage/transport space via the air inlet opening into the flow channel inside the air purifier and to blow purified air via the air outlet opening back into the storage/transport space, a filter system arranged in the flow channel in such a manner that the air flows through the filter system, wherein the filter system comprises, as chemical filter material, both a UV-activated photocatalyst and a chemical filter material comprising potassium permanganate, and a UV-emitting light source for activating the photocatalyst of the filter system. The filter system comprises a main filter, which forms a cassette that comprises at least two crates, wherein a first crate contains potassium permanganate and a second crate contains a selected chemical filter material, and wherein the air is made to flow through the crates.

The system according to the invention is designed to rid air of ethylene produced in particular by plants present in the space. In addition, the system effectively eliminates bacteria, viruses, mould and VOC gases from the air. By combining a plurality of chemical filter materials in the air purifier, different air contaminants can be eliminated very effectively. By combining potassium permanganate with a UV-activated photocatalyst in the same air purifier, the rate of ethylene removal increases significantly, and is even multiplied, compared to a photocatalyst alone. Potassium permanganate, especially when used together with UV-activated titanium dioxide, degrades ethylene in the air very effectively. The accommodation of the potassium permanganate in a crate makes it possible to use potassium permanganate bound to pellets made of, for example, aluminium oxide as the filter material. The fact that the main filter of the filter system comprises crates in which a selected filter material is accommodated makes the handling of the filter materials easier and the filter system can be readily dimensioned according to the requirements of a storage/transport space and the produce to be treated in that space. By using at least two crates in the main filter, it becomes possible to employ at least two different filter materials in the main filter or to readily adjust the amount of only the potassium permanganate.

The design of the main filter as a cassette structure comprising removable crates thus makes it possible to readily adapt the air purifier to usage requirements as well as to use, as a function of the space, different amounts of filter materials and filter materials comprising different active substances. By means of the crates of the main filter, it is possible to employ solid filter materials, in particular a filter medium, in the main filter. A system with at least two crates in series through which the air is made to flow permits a multiplicative action, so that contaminants in the air can be eliminated more effectively than to date.

According to the invention, the system further comprises an ethylene sensor that measures an ethylene concentration in the air of the storage/transport space. By means of a continuous monitoring of the ethylene concentration, a storage/transport space can be ventilated as needed or the removal of ethylene can be increased if the ethylene concentration becomes too high so as to pose a potential risk to stored produce.

Advantageously, the system further comprises data transmission means for sending measurement data generated by the ethylene sensor to a server, and recording means for recording the measurement data. It is thus possible to form log data of conditions in the storage/transport space for a selected time period.

It is also possible to measure, transmit and record levels of humidity, temperature, carbon dioxide, as well as of VOCs and fine particles, such as PM2.5 and PM10, in the storage/transport space by means of appropriate sensors. Conditions in the storage/transport space can thus be monitored in even greater detail in order to maintain optimal conditions.

Advantageously, the main filter further comprises at least one fabric filter, which advantageously comprises a selected chemical filter material. This permits an increase in the performance of the filter system and/or the use of filter materials in the fabric filter that it is not possible to place or that are poorly suited to being placed in a crate. With fabric filters it becomes possible to use filtering nonwoven fabrics in the main filter. The fabric filter can also filter filter material accommodated in a crate so as to prevent the filter material from ending up outside the air purifier, whereby filter material potentially harmful to humans or produce in the space is prevented from ending up in unwanted places.

Advantageously, the crates can be removed from the cassette. This allows the crates and/or the filter material accommodated in the same to be readily replaced in the main filter.

Advantageously, the main filter comprises 2-8 crates, advantageously 4-6 crates. It is thus easy to provide a desired amount of a selected filter material in the main filter.

Advantageously, the main filter of the air purifier includes at least one crate in which the accommodated filter material comprises a UV-activated photocatalyst, advantageously glass tubes coated with titanium dioxide. The main filter thereby purifies the air effectively of ethylene and other organic substances, bacteria, viruses, mould, and VOC gases.

The main filter of the air purifier can have a cover made of a highly light-permeable plastic, advantageously of polymethyl methacrylate, advantageously of a thickness of 2-4 mm, more advantageously of a thickness of 2-3 **mm.** The photocatalytic filter material accommodated in the main filter can thus be activated by means of a light source outside the main filter.

This allows a light source to be arranged in a fixed manner inside the air purifier and the main filter to be replaced without removing the light source.

The main filter of the air purifier can comprise a fabric filter that comprises activated carbon, and which is advantageously the last filter in the filter system in the direction of air flow. This makes it possible to remove gases and bad smells from the air. In addition, an activated carbon filter arranged last in the direction of flow ensures that the potassium permanganate and other chemical filter materials cannot be transported via the air outlet opening to the outside of the air purifier.

Advantageously, the UV-activated photocatalyst of the air purifier is titanium dioxide, copper oxide or zinc oxide. These photocatalysts have proven to be effective in the purification of air in storage/transport spaces.

Advantageously, the filter system of the air purifier also comprises activated carbon and/or nanosilver as filter material. This makes it possible to effectively remove a plurality of different contaminants from the air besides ethylene.

The air purifier can also comprise a HEPA filter for removing airborne particles from the storage/transport space.

Advantageously, the UV-emitting light source of the air purifier is a UVC-emitting LED with a wavelength of advantageously 255-275 nm. Using LEDs as the light source allows the same light source to last the lifetime of the air purifier and makes the air purifier very energy efficient. At this wavelength, the photocatalytic properties of in particular titanium dioxide are significantly enhanced.

Advantageously, the air purifier comprises an auxiliary light source, which is advantageously a blue light emitting LED. The blue LED thus produces visible light that can be used as an indicator to a user that the air purifier is in operation. To this end, the auxiliary light source can be connected to the same electric circuit as the light source mentioned above. In addition, the blue LED acts as an additional catalyst for the photocatalytic filter material, so that the operation of the filter system is more effective.

Advantageously, the main filter of the air purifier is fixed in the flow channel with fixation means in such a manner that the main filter can be removed without tools. The filter materials accommodated in the main filter are subject to wear during use, whereby their effectiveness declines. In cases where the system is to be used continuously, it is essential that the filter materials are replaced regularly. It can also be desirable for the amount and/or quality of the filter materials accommodated in a crate to be changed as the need arises, so that it can be necessary to remove the main filter frequently. In this case, these actions can be carried out quickly and easily, as the main filter is fixed in the air purifier in such a manner that its removal does not require tools. This makes the maintenance of the air purifier very easy. Advantageously, the main filter of the air purifier can be replaced in less than a minute without tools.

According to the invention, the main filter of the air purifier is arranged inside the casing by means of slide rails in such a manner that the main filter can be removed without opening the casing. This makes it easy to remove and replace the main filter, as it is not necessary to open the casing of the air purifier in order to remove the main filter.

In an embodiment of the invention, the casing of the air purifier can also be closed by means of a quick-release fastener in such a manner that the casing can be readily opened without tools, and the replacement of the main filter can require the opening of the casing.

Advantageously, the filter system of the air purifier further comprises a pre-filter arranged in the flow channel, wherein the pre-filter comprises a nonwoven fabric that comprises a selected chemical filter material. This allows the overall performance of the air purifier to be improved.

Advantageously, the pre-filter is arranged in the air flow channel upstream of the main filter in the direction of air flow. This allows the overall performance of the air purifier to be improved.

The nonwoven fabric of the pre-filter of the air purifier can comprise a UV-activated photocatalyst, advantageously titanium dioxide, and the UV-emitting light source can be directed at the pre-filter. The air sucked into the flow channel can thus be effectively pre-filtered before being conducted to the main filter, so that the overall air-purifying performance of the system is improved. This also makes it easy to direct the light source at the photocatalyst in order to activate the photocatalyst. The pre-filter thus effectively purifies the air of ethylene and other organic substances, bacteria, viruses, mould, and VOC gases. The nonwoven fabric of the pre-filter can further comprise nanosilver. In addition to nonwoven fabric, it is also possible to use alternative materials, such as aluminium oxide or glass, as structural materials in the pre-filter.

Advantageously, the pre-filter of the air purifier is fixed in the flow channel with fixation means in such a manner that the pre-filter can be removed without tools. This makes a removal and replacement of the pre-filter easy and fast in the context of a maintenance of the system, which reduces maintenance costs.

Advantageously, the pre-filter of the air purifier is arranged inside the casing by means of slide rails so that the pre-filter can be removed without opening the casing. This makes it easy to remove and replace the pre-filter. Alternatively, the methods for fixating the main filter mentioned above and known *per se* can be used for the pre-filter.

Advantageously, the air purifier also comprises a coarse filter, which prevents macroscopic particles from entering the air flow channel.

The system advantageously includes software means configured to trigger an alarm if an ethylene concentration exceeds a pre-defined threshold value. An increase in an ethylene concentration above a threshold value is thus known quickly, which makes it possible to immediately initiate the required measures for the prevention of a spoiling of produce in the storage or transport space.

Advantageously, the system includes software means for reading both a real-time ethylene concentration and a parameter which describes the operation of the air purifier and which is added to the measurement data. This makes it possible to continuously monitor the conditions of the air in the storage or transport space. A user is thus also constantly informed of the action of the air purifier.

The system is advantageously configured to measure and record the ethylene concentration in a database in intervals of 1 s - 10 min, advantageously in intervals of 10 s - 2 min. Changes in the ethylene concentration are thus captured with sufficient precision.

Advantageously, the storage/transport space comprises an air-circulating refrigeration machine, and the air purifier is arranged on a ceiling of the storage/transport space near the refrigeration machine, and the refrigeration machine of the storage or transport space is configured to supply the air purifier with power. A device thus does not take space away from the produce to be stored or transported and the air purifier does not need a separate power source, but rather can draw its operating power from the refrigeration machine. An air purifier in a storage space can also be connected to an electrical grid.

The nominal volume of the storage/transport space is advantageously 5 - 500 m³, most advantageously 50 - 250 m³. In this range, the air purifier is suitable in terms of its performance for the purification of the air in the storage/transport space. The crate structure of the main filter, however, allows a very good scaling of the system according to the invention to spaces of different sizes and for different applications, since the active agent of the filter material and its amount can be easily adjusted.

Advantageously, the effective air volume of the storage or transport space is 5 - 60%, advantageously 8 - 30%, of the nominal purification capacity of the air purifier. A surprisingly large transport/storage space can thus be purified using a low-power air purifier. The effective air volume may be considerably smaller than the nominal volume if the goods present in the space take up a considerable part of the total volume.

The nominal air flow of the fan of the air purifier can be 100 - 300 m³/h. This allows the air flow flowing through the air purifier to reach a desired level in order to purify the air in a closed storage/transport space effectively. An effectiveness of the air purifier with respect to the flow rate here means that the air must flow through the air purifier slowly enough to allow contaminants enough time to react with the chemical filter materials in order to eliminate the contaminants, yet fast enough to permit the treatment of the entire effective air volume of the storage or transport space in a sufficient time interval. Advantageously, the flow rate of the air purifier is adjusted as a function of the effective air volume of the available space, the plants in the space and the filter materials employed.

Advantageously, the system comprises software means for regulating a fan power and/or for starting the fan when the ethylene concentration measured by the ethylene sensor exceeds a predetermined threshold value. The rate of air flow in the flow channel of the air purifier can thus be regulated according to existing conditions. For example, the fan can be kept at a low power in order to reduce energy consumption and noise when a need for air purification is lower, and the power can be increased automatically as needed.

The invention is illustrated in the following in detail with reference to the attached drawings illustrating embodiments of the invention, wherein
- Figure 1a: illustrates an axonometric projection of an air purifier according to the invention in an external view from the front,
- Figure 1b: illustrates an axonometric projection of an air purifier according to the invention in an external view from the rear,
- Figure 2: illustrates an opened air purifier according to the invention,
- Figure 3a: illustrates a pre-filter of an air purifier according to the invention,
- Figure 3b: illustrates an opened pre-filter of an air purifier according to the invention,
- Figure 4a: illustrates a main filter of an air purifier according to the invention,
- Figure 4b: illustrates an opened main filter of an air purifier according to the invention,
- Figure 5: schematically illustrates a system according to the invention in a transport space,
- Figure 6: shows measurement data relating to the effect of the system according to the invention on the ethylene concentration in a closed space.

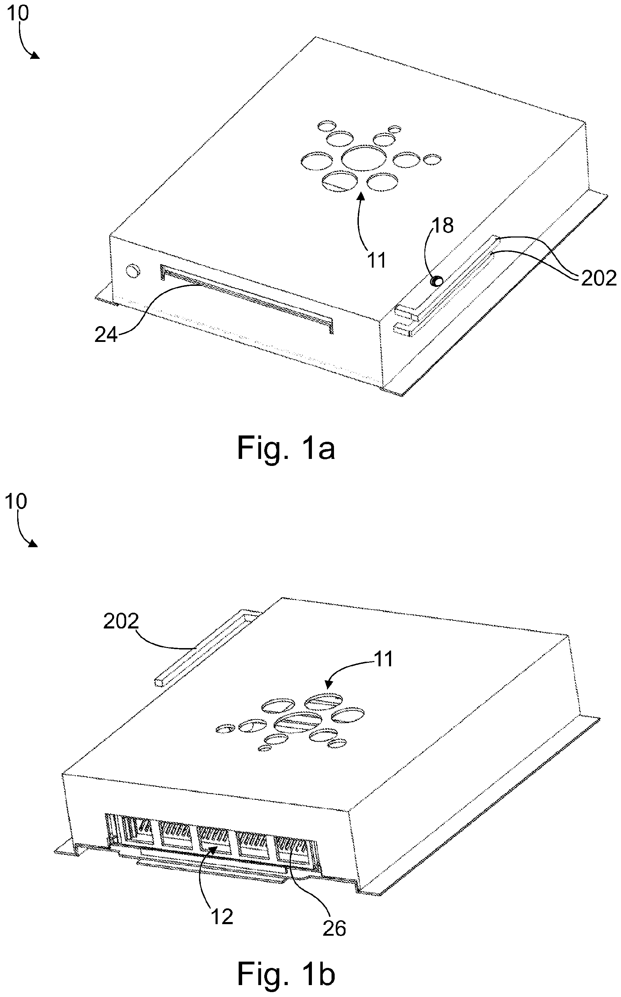

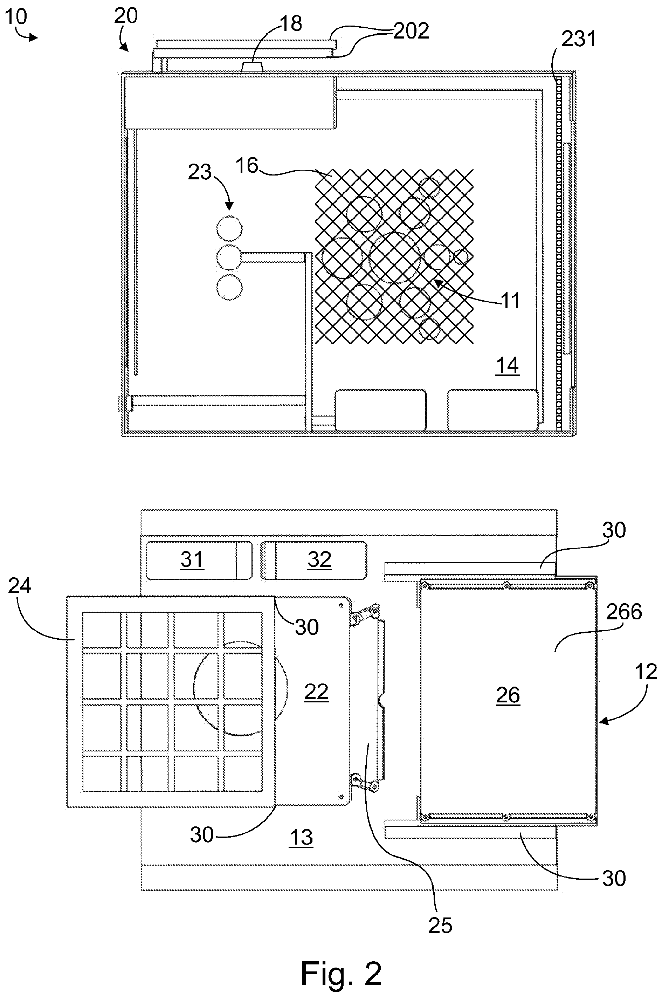

Figures 1a and 1b illustrate an air purifier 10 according to the invention in an external view. Figure 2 illustrates an opened air purifier 10, so that the parts inside the casing are visible. The casing of the air purifier 10 consists of a base plate 13 and a cover plate 14, to which the other parts of the air purifier 10 are attached, and between which an air flow channel is formed when the casing is closed.

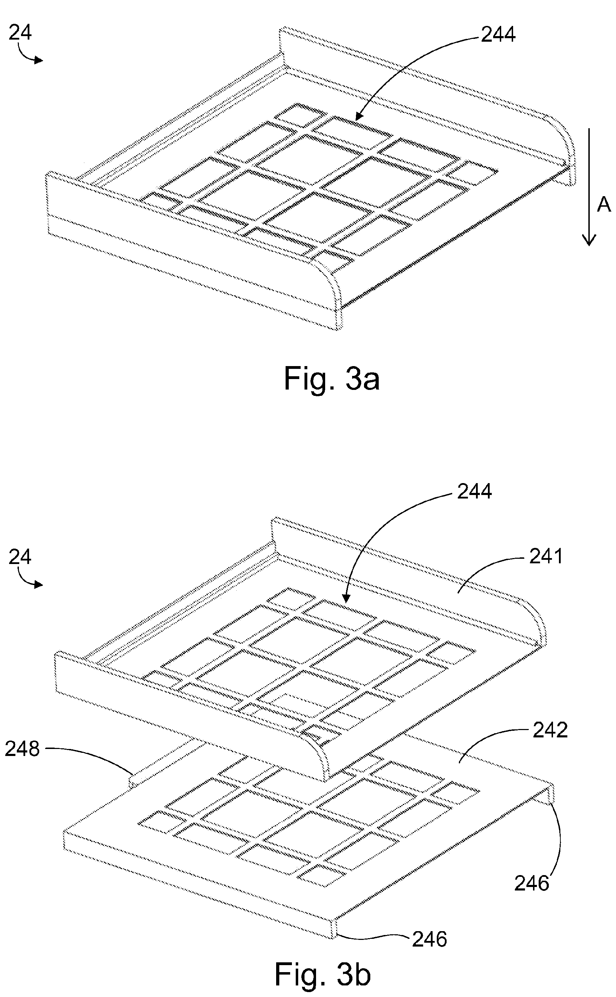

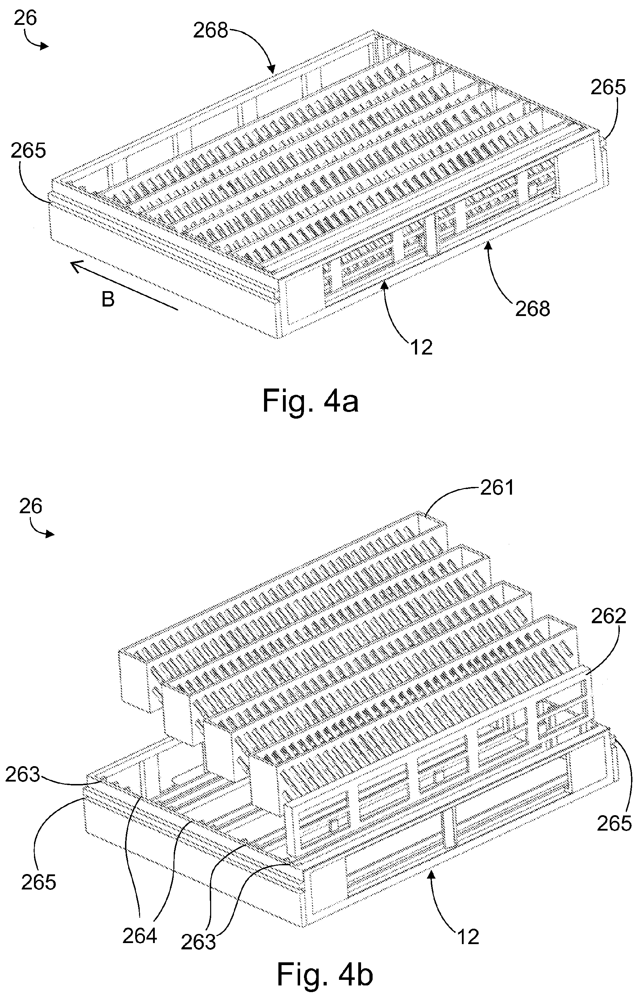

An air inlet opening 11 and an air outlet opening 12, both of which consisting of a plurality of individual openings in this embodiment, are visible in the exterior view of the air purifier 10. Lying inside the casing between the air inlet opening 11 and the air outlet opening 12 is an air flow channel containing a filter system. The flow channel includes a fan 22, which is configured to suck air via the air inlet opening 11 into the flow channel inside the air purifier 10. A coarse filter 16 is arranged in the air inlet opening 11, wherein all air entering the flow channel flows first through the coarse filter 16. The coarse filter 16 prevents macroscopic particles from entering the air filter 10. After the coarse filter 16, the air passes through a pre-filter 24 in the flow channel. The structure of the pre-filter 24 is explained in greater detail in the description later on (Figures 3a and 3b). The pre-filter 24 is arranged over the fan 22 and is the same size as the fan 22 in terms of its surface area, so that all the air flowing in the flow channel passes through the pre-filter 24. The fan 22 is thus downstream of the pre-filter 24 in the flow channel, between the pre-filter 24 and the main filter 26. From the fan 22, the air flows through an air guide 25 to a main filter 26, the structure of which is also explained in greater detail in the description later on (Figures 4a and 4b). The rear wall of the main filter 26 forms the air outlet opening 12 of the air purifier 10, via which purified air that has also passed through the main filter 26 exits the air purifier 10.

Both the pre-filter 24 and the main filter 26 are arranged inside the air purifier 10 by means of slide rails 30. More specifically, the base plate 13 of the air purifier 10 is designed to include first slide rails 30 in which the main filter 26 is arranged in such a manner that the pre-filter 24 is pushed through a side wall of the air purifier 10 into the air purifier 10. The pre-filter 24, on the other hand, is designed in this embodiment so as to slide over the fan 22 through the opposite side wall of the air filter 10. The opening in the cover plate 14 for the pre-filter 24 is configured in such a manner that the upper part 241 of the pre-filter 24 rests against the cover plate 14 when inside the air purifier 10. The pre-filter 24 is thus pressed tightly against the fan 22. The inner edges of the lower part 242 of the pre-filter 24 lie outside the edges of the fan 22, which prevents a lateral movement of the pre-filter 24. An edge of both the pre-filter 24 and the main filter 26 remains in the plane of the wall of the air purifier 10 or advantageously just outside of it, whereby it is easy to grab hold of the filters from outside the air purifier and both filters can be pulled out of the air purifier 10 without tools and replaced with new ones very quickly. This can occur without opening the casing of the air purifier 10, i.e. without separating the cover plate 14 from the base plate 13. In Figure 2, the pre-filter 24 and the main filter 26 are partially pulled on the slide rails 30 out of the casing of the air purifier 10 to illustrate the mechanical movement of the pre-filter 24 and the main filter 26 during installation and removal. The end of the slide rails 30 advantageously includes a small clasp which locks the main filter 26 and/or the pre-filter 24 in place inside the casing.

In this embodiment, the system according to the invention further comprises an ethylene sensor 18 that measures the ethylene concentration. In this embodiment, the ethylene sensor 18 is arranged on a side wall of the air purifier 10 in such a manner that the ethylene sensor measures an ethylene concentration of the air outside the air purifier 10. Alternatively, the ethylene sensor 18 can be arranged inside the air purifier 10 near the air inlet opening 11 in the air flow channel, upstream of the pre-filter 24 in the direction of air flow. With respect to its arrangement, it is essential that the ethylene sensor 18 is arranged so as to measure the actual ethylene concentration of the air in the space, and not the ethylene concentration of the air flowing out of the air purifier 10. More specifically, in this case (Figure 1a), only the active measuring end of the ethylene sensor 18 is outside the casing of the air purifier 10, the rest of the measurement electronics being housed inside the casing of the air purifier 10. Alternatively, the ethylene sensor 18 and its apparatus can also be arranged in the form of a separate unit outside the air purifier 10. In this case, it is protected by a housing and power can be drawn from inside the air purifier 10.

The ethylene sensor 18 can be, for example, a Membrapor C2H4/M-200 model, which can measure an ethylene concentration with a resolution of 1 ppm in the range 0 - 200 ppm. Examples of circuit connections of an ethylene sensor 18 can be found in the publications
- Membrapor, "Specification Sheet for Ethylene Sensor Type C2H4/M-200",
- Membrapor, "4 to 20 mA Transmitter Board for Electrochemical Gas Sensor Suitable for Compact-Sensors (Pin layout: 7-Series)",
- CO2Meter, Inc., "EC200 Electrochemical Sensor Controller Manual", and
- N.A. Zaidi et al., "A Gas Chromatographic System for the Detection of Ethylene Gas Using Ambient Air as a Carrier Gas", Sensors 2017, 17, 2283.

The ethylene sensor 18 is connected to data transmission means 20, which include a router and antennas 202. The ethylene concentration can be measured, for example, in 30 second intervals and the measurement data transmitted by the data transmission means 20 to a server via a mobile phone network. This makes it possible to monitor the ethylene concentration of the space in real time via a remote connection and to record the historical data of the ethylene concentration over long time intervals. In addition to an ethylene concentration, it is also possible to send a parameter that describes the operation of the air purifier 10 together with the measurement data to the server via the data transmission means 20, wherein said parameter conveys a status of the air purifier to a user.

In this embodiment, two antennas 202 are arranged on the air purifier 10, one operating on a GSM network suitable for data transmission over long distances and the other operating on a WLAN network suitable for local data transmission.

In this embodiment, the UV-activated photocatalyst is arranged in the pre-filter, for which a UV-emitting light source 23 is arranged on the cover plate 14 of the air filter 10. More specifically, the light source 23 consists of UVC-emitting LEDs, three in this embodiment, whose emitted radiation has a peak intensity of 270 **nm.** In another embodiment, two 275 nm UVC LEDs are used as the light source with a current of 100 **mA.**

In addition, the air purifier 10 in this embodiment also comprises an auxiliary light source 231, which is a blue LED here. The blue LED is connected to the same electric circuit as the UVC LEDs, which do not produce visible light. The blue LED thus acts as an indicator to a user that the air purifier 10 is in operation. In addition, the blue LED acts as an auxiliary catalyst for the photocatalytic filter material, which improves the performance of the filter.

The air purifier 10 can be configured to use an electric grid or a selected lower voltage as its power source as a function of the application. In this embodiment, the air purifier 10 uses electricity from a grid, so that the air purifier 10 includes a first transformer 31 by means of which the voltage is adapted to the fan 22, which uses a voltage of 24 V. The air purifier 10 further includes a second transformer 32 by means of which the voltage is adapted to the light source 23 as well as to the measurement apparatus comprising the ethylene sensor 18 and the data transmission means 20, which use a voltage of 12 V.

In this embodiment, the fan 22 is a radial fan of the model RG160-28/14N with a wattage of 20 W and a nominal air flow of 209 m³/h.

Figures 3a and 3b illustrate a pre-filter 24 of an air purifier 10 according to the invention, which consists of a plastic frame in which a nonwoven fabric is arranged. More specifically, the pre-filter 24 here comprises an upper part 241 and a lower part 242, between which a nonwoven fabric is interposed that is not illustrated in the figures. The flow channel of the air purifier 10 runs through the pre-filter 24, through the openings 244 in the pre-filter and thus through the nonwoven fabric arranged in the pre-filter 24. The pre-filter 24 here comprises projections 246 by means of which the pre-filter 24 is supported on top of the fan 22, the edges of the fan 22 forming slide rails 30. In addition, the pre-filter 24 illustrated in Figure 3 has a gripping part 248 that remains outside the air purifier 10 when the pre-filter 24 is inserted inside the air purifier 10. A user can thus grip the gripping part 248 and pull the pre-filter by the gripping part 248 out of the air purifier 10 in order to replace the pre-filter 24. The air flows inside the air purifier 10 through the pre-filter 24 in the direction indicated by the arrow **A.**

In this embodiment, the nonwoven fabric of the pre-filter 24 is treated by immersion in a solution containing titanium dioxide, nanosilver and water. Titanium dioxide and nanosilver remain in the dried nonwoven fabric and together cause the oxidation of any inorganic substances in the air into water and carbon dioxide without producing ozone. The oxidation reaction can be accelerated by UV radiation. The UVC LEDs acting as the light source 23 and the blue LED acting as the auxiliary light source 231 are thus directed at the pre-filter. With the pre-filter 24, bacteria, viruses, mould and in part VOC gases can be eliminated from the air.

Instead of or in addition to titanium dioxide, it is also possible to use copper oxide or zinc oxide, which are also photocatalysts.

Figures 4a and 4b illustrate a main filter 26 without a cover 266. The main filter 26 forms a cassette in which a selected amount of selected chemical filter materials can be placed in either crates 261 or fabric filters 262. The inner walls of the cassette structure of the main filter 26 contain wide slots 264, in which crates 261 can be fixed, and narrow slots 263, in which fabric filters 262 can be fixed. In this embodiment, a maximum of four crates 261 and five fabric filters 262 can be arranged in the main filter 26. Fabric filters 262 can be placed before and after the crates 261 as well as between crates 261. It is also possible to leave positions for crates 261 and fabric filters 262 unused as a function of application requirements. For example, in Figures 4a and 4b, the last position for a fabric filter 262 in the direction of air flow is left empty, as are the positions for fabric filters 262 between the crates 261. Inside the air purifier 10, the air flows through the main filter 26, through the openings 268 in the opposite side walls of the main filter 26, in the direction indicated by the arrow B, successively passing through all crates 261 and fabric filters 262 located in the main filter 26. The crates 261 and fabric filters 262 also have openings through which the air flow passes, which thus also passes through the chemical filter materials accommodated in the crates 261 and fabric filters 262. The fabric filters 262 are formed in the same manner as the pre-filter 24, i.e. a nonwoven fabric comprising an active chemical filter material is arranged interposed between two parts forming a plastic frame. In terms of its structure, a chemical filter material accommodated in the crates 261 can be in any solid form, such as granules, and an active agent can be bound to a selected structural material. The positions for fabric filters 262 here are first and last in the direction of air flow, so that the crates 261 are positioned between the fabric filters 262, as well as between the crates 261. The crates 261 and the fabric filters 262 can also be positioned differently in relation to one another. The outer surfaces of the side walls of the main filter 26 include corresponding elements 265 for the slide rails 30, by means of which the main filter 26 can be slid into the air purifier 10.

The removal of ethylene is important, for example, in the transport and storage of fresh fruit and vegetables, which is why potassium permanganate is used in the main filter 26. The filter material to be accommodated in a selected crate 261 can be, for example, Climecon CKP 8 filter medium, which comprises aluminium oxide as structural material and whose potassium permanganate concentration constitutes at least 8 % of the weight of the filter medium.

The filter medium to be accommodated in a selected crate 261 can also be, for example, activated carbon, such as the Climecon adsorption medium ECS, or a mixture of potassium permanganate and activated carbon, such as the filter medium Climecon CEKP 8. Activated carbon effectively rids air of various gases, such as sulphur gas.

A selected crate 261 can accommodate, for example, glass tubes coated with titanium dioxide, which remove gases, bacteria, and viruses. Titanium dioxide requires UV radiation in order to accelerate the reaction. Consequently, in cases where a photocatalyst is used in the main filter, UV radiation is provided to the main filter. The glass tubes can have, for example, an outer diameter of 4 mm, an aperture diameter of 2 mm and a length of 20 **mm.** Alternatively, different materials, such as aluminium oxide, can be used as the structural material of a photocatalyst such as titanium dioxide.

After the crates 261, a fabric filter 262 comprising activated carbon is advantageously placed last in the direction of air flow. This filter removes, *inter alia,* VOC gases and bad smells. In addition, an activated carbon filter arranged last in the direction of flow ensures that potassium permanganate and other chemical filter materials cannot be transported via the air outlet opening 12 to the outside of the air purifier 10.

The air purifier 10 according to the invention has been extensively tested using a Climecon CKP 8 filter medium with a total weight of 300 g in the main filter 26. The potassium permanganate content is 8% by weight, i.e. there is thus altogether approximately 24 g of potassium permanganate in the main filter 26. The mass of the filter medium was measured before and after each test during which the air purifier was used for 3-4 hours at a time. There was no decrease in the mass of the filter medium of the main filter 26 during the tests, which rather increased due to the accumulation of moisture in the filter medium of the main filter 26. It can be estimated that moisture also helps to bind the potassium permanganate. Small amounts of potassium permanganate may still break off from the surface of the filter medium, but this potassium permanganate cannot spread to the outside of the air purifier, because any potassium permanganate having broken off from the filter medium is stopped at the latest by the filter arranged last in the main filter 26, the tight fabric filter 262 comprising activated carbon.

The filters of the air purifier 10 are typically replaced in intervals of approximately 4-6 months, depending on usage.

The amount of filter material can be dimensioned, for example, in such a manner that, where Climecon CKP 8 filter material is used in a 200 m³ storage/transport space, 300 g of filter material is used in the main filter 26. If the volume is tripled to 200 m³, the mass of the filter medium is doubled to 600 g. The crate structure of the main filter 26 makes it very easy to modify an amount of filter material.

In dry spaces, a fabric filter 262 with a nonwoven fabric comprising titanium dioxide and nanosilver on one side and a nonwoven fabric comprising activated carbon on the other side can also be used in the main filter 26. A plurality of these fabric filters 262 can also be arranged in series as needed.

The number of crates 261 and fabric filters 262 used in the main filter 26 is selected according to the air volume of the space, the plants stored in the space and the desired fan speed.

The top of the main filter 26 comprises a cover 266, which is visible as the uppermost part of the main filter 26 in Figure 2. The cover 266 locks the crates 261 and the fabric filters 262 inside the main filter 26. In cases where a photocatalyst requiring UV radiation is used in the main filter, the cover 266 can be made of a highly light-permeable plastic. The cover 266 of the main filter 26 can be made of, for example, polymethyl methacrylate, which has a permeability for UV radiation at a thickness of 3 mm of approximately 55% where a wavelength of 270 nm is employed. A 2mm thickness of the cover 266 suffices to achieve structural strength, whereby the permeability increases to approximately 70% if polymethyl methacrylate is used, and to up to 85% with some materials.

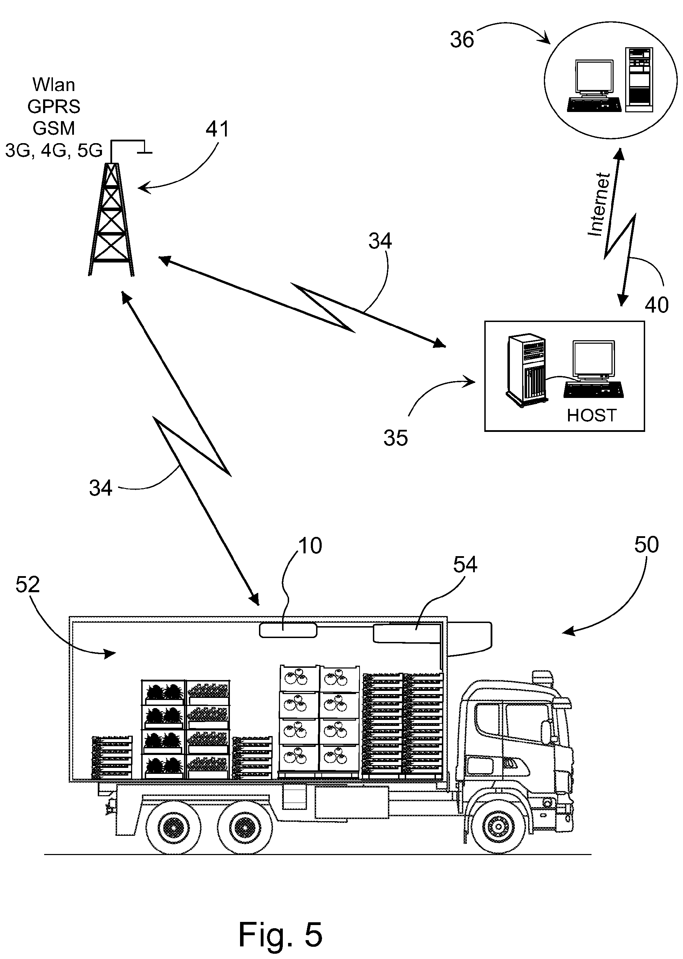

In the situation illustrated in Figure 5, an air purifier 10 is arranged in a transport space 52 of a truck 50. The transport space 52 contains an air-circulating refrigeration machine 54 by means of which the transport space 52 is maintained at a selected temperature during transport in order to improve preservation of the plants. The air purifier 10 is arranged near the refrigeration apparatus 54 on the ceiling of the transport space 52, so that it does not take space away from the load. The air purifier 10 is configured to draw its operational energy from the refrigeration machine 54.

During transport, the ethylene concentration of the transport space 52 can be monitored in real time. The reading of the ethylene sensor 18 is sent by data transmission means 20 via a network 34 to a server 35 in selected intervals, for example in 30 second intervals. The ethylene concentration of the transport space 52 can be recorded in a memory by recording means provided on the server 35. The server 35 can be accessed with a computer 36 via the internet 40. The computer 36 can be, for example, a mobile phone of the driver of the truck 50. The same server 35 can serve a plurality of different transport and storage spaces.

The network 34 can be any data communications network. Different options are, for example, GSM, GPRS, 3G, 4G, 5G and WLAN. The connection between the base stations 41 and the server 35 is naturally operator-specific and is of no significance from the point of view of the present invention.

By means of software means provided on the server 35, the system can be configured to trigger an alarm if the ethylene concentration in the transport space 52 exceeds a pre-defined threshold value, for example 20 ppm. The threshold value can be exceeded, for example, if the air purifier 10 removing the ethylene fails. An alarm can also be triggered if a parameter sent to the server that describes the operation of the air purifier 10 indicates a device failure. For example, if the ethylene concentration exceeds a threshold value during the transportation of plants or if the air purifier 10 fails, an alarm can be transmitted to the mobile phone of the driver of the truck 50, so that the transport space 52 can be ventilated immediately to reduce the ethylene concentration or so that the operation of the air purifier 10 can be checked. Alternatively, an automated ventilation can be provided in the transport space 52, which turns itself on if the ethylene concentration exceeds a threshold value.

The air purifier 10 can also be used in plant storage spaces, for example, in refrigerated spaces of stores where fruit, vegetables and flowers await sale.

The system according to the invention allows, for example, tomatoes and flowers to be stored and transported in the same space, which would not be possible without an effective air purification due to the accumulation of ethylene.

Although the system according to the invention is mainly aimed at applications for foodstuffs, it is clear that the use of the system is not limited to the illustrated examples but can be used to purify the air in any space using selected filter materials.

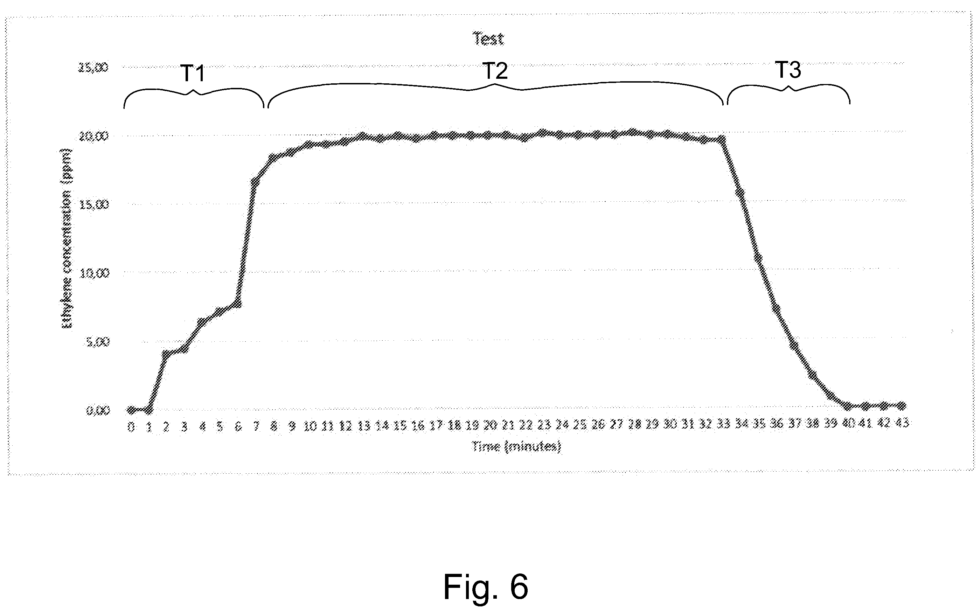

Figure 6 shows the ethylene concentration of a closed space as a function of time. Ethylene was removed from a 28*38*58 cm sized space by means of a system according to the invention, in which potassium permanganate, titanium dioxide and nanosilver were used as the active ingredients of the filter materials for the removal of ethylene. More specifically, in the system used in the test depicted in Figure 6, the main filter contained 300 g of Climecon CKP 8 filter medium comprising potassium permanganate and the pre-filter comprised UV-activated titanium dioxide and nanosilver. Initially, a gas mixture containing ethylene was introduced into the space and the ethylene concentration in the space was increased to 20 ppm in the time interval T1, i.e. in the time interval 0-7 min. The introduction of ethylene into the space was then stopped and the space was left to stabilize in the time interval T2, i.e. in the time interval 7-33 min. At 33 min, the air purifier 10 according to the invention was turned on and, in the time interval T3, i.e. in the time interval 33-40 min, it was possible to rid the space entirely of ethylene. Based on measurements, the air purifier 10 according to the invention is able to remove ethylene at a rate of 3 ppm/min, which is many times higher than, for example, an air purifier that uses titanium dioxide alone.

## Claims

1. A system for purifying air in a closed storage/transport space, which system comprises an air purifier (10) comprising
- a casing with an air inlet opening (11) and an air outlet opening (12), wherein an air flow channel lies between the air inlet opening (11) and the air outlet opening (12) inside the casing,
- a fan (22) configured to suck air from the storage/transport space via the air inlet opening (11) into the flow channel inside the air purifier (10) and to blow purified air via the air outlet opening (12) back into the storage/transport space,
- a filter system arranged in the flow channel in such a manner that air flows through the filter system, wherein the filter system comprises, as chemical filter material, both a UV-activated photocatalyst and a chemical filter material comprising potassium permanganate, and
- a UV-emitting light source (23) for activating the photocatalyst of the filter system,
- the filter system comprises a main filter (26), which forms a cassette that comprises at least two crates (261), wherein the filter material accommodated in the first crate (261) contains potassium permanganate and the second crate (261) contains a selected chemical filter material, and wherein air is made to flow through the crates (261),
**characterized in that**
- the system further comprises an ethylene sensor that measures an ethylene concentration in the air of the storage/transport space (18), and
- the main filter (26) of the air purifier (10) is arranged inside the casing by means of slide rails (30) in such a manner that the main filter (26) can be removed without opening the casing.

2. The system according to claim 1, **characterized in that** the system further comprises data transmission means (20) for sending measurement data generated by the ethylene sensor (18) to a server, and recording means for recording the measurement data.

3. The system according to claim 1 or 2, **characterized in that** the main filter (26) further comprises at least one fabric filter (262), which advantageously comprises a selected chemical filter material.

4. The system according to any of claims 1 - 3, **characterized in that** the main filter (26) of the air purifier (10) includes at least one crate (261) in which the accommodated filter material comprises a UV-activated photocatalyst, advantageously glass tubes coated with titanium dioxide.

5. The system according to any of claims 1 - 4, **characterized in that** the main filter (26) of the air purifier (10) comprises a fabric filter (262) that comprises activated carbon, and which is advantageously the last filter of the filter system in the direction of air flow.

6. The system according to any of claims 1 - 5, **characterized in that** the UV-activated photocatalyst of the air purifier (10) is titanium dioxide, copper oxide or zinc oxide.

7. The system according to any of claims 1 - 6, **characterized in that** the filter system of the air purifier (10) also comprises activated carbon and/or nanosilver as filter material.

8. The system according to any of claims 1 - 7, **characterized in that** the UV-emitting light source (23) of the air purifier (10) is a UVC-emitting LED with a wavelength of advantageously 255-275 nm.

9. The system according to claims 1 - 8, **characterized in that** the filter system of the air purifier (10) further comprises a pre-filter (24) arranged in the flow channel, wherein the pre-filter (24) comprises a nonwoven fabric that comprises a selected chemical filter material.

10. The system according to claim 9, **characterized in that** the nonwoven fabric of the pre-filter (24) of the air purifier (10) comprises a UV-activated photocatalyst, advantageously titanium dioxide, and a UV-emitting light source (23) is directed at the pre-filter (24).

11. The system according to claim 9 or 10, **characterized in that** the pre-filter (24) of the air purifier (10) is arranged inside the casing by means of slide rails (30) in such a manner that the pre-filter (24) can be removed without opening the casing.

12. The system according to any of claims 1 - 11, **characterized in that** the system includes software means configured to trigger an alarm if an ethylene concentration measured by the ethylene sensor (18) exceeds a pre-defined threshold value.

13. The system according to any of claims 1 - 12, **characterized in that** the system includes software means for reading both a real-time ethylene concentration and a parameter which describes the operation of the air purifier and which is added to the measurement data.

14. The system according to any of claims 1 - 13, **characterized in that** the storage/transport space comprises an air-circulating refrigeration machine (54), and the air purifier (10) is arranged on a ceiling of the storage/transport space near the refrigeration machine (54), and the refrigeration machine (54) of the storage or transport space is configured to supply the air purifier (10) with power.

15. The system according to any of claims 1 - 14, **characterized in that** the system comprises software means for regulating a power of the fan (22) and/or for starting the fan (22) when the ethylene concentration measured by the ethylene sensor (18) exceeds a predetermined threshold value.

## Patentansprüche

1. System zur Reinigung von Luft in einem geschlossenen Speicher-/Transportraum, wobei das System einen Luftreiniger (10) umfasst, umfassend:
- ein Gehäuse mit einer Lufteinlassöffnung (11) und einer Luftauslassöffnung (12), wobei zwischen der Lufteinlassöffnung (11) und der Luftauslassöffnung (12) innerhalb des Gehäuses ein Luftströmungskanal liegt,
- einen Ventilator (22), der so konfiguriert ist, dass er Luft aus dem Speicher-/Transportraum über die Lufteinlassöffnung (11) in den Strömungskanal innerhalb des Luftreinigers (10) saugt und gereinigte Luft über die Luftauslassöffnung (12) zurück in den Speicher-/Transportraum bläst,
- ein Filtersystem, das in dem Strömungskanal so angeordnet ist, dass Luft durch das Filtersystem strömt, wobei das Filtersystem als chemisches Filtermaterial sowohl einen UV-aktivierten Photokatalysator als auch ein chemisches Filtermaterial umfasst, das Kaliumpermanganat umfasst, und
- eine UV-emittierende Lichtquelle (23) zum Aktivieren des Photokatalysators des Filtersystems,
- wobei das Filtersystem einen Hauptfilter (26) umfasst, der eine Kassette bildet, die mindestens zwei Kästen (261) umfasst, wobei das in dem ersten Kasten (261) untergebrachte Filtermaterial Kaliumpermanganat enthält und der zweite Kasten (261) ein ausgewähltes chemisches Filtermaterial enthält, und wobei Luft durch die Kästen (261) strömen gelassen wird,
**dadurch gekennzeichnet, dass**
- das System ferner einen Ethylensensor umfasst, der die Ethylenkonzentration in der Luft des Speicher-/Transportraums (18) misst, und
- der Hauptfilter (26) des Luftreinigers (10) mittels Gleitschienen (30) innerhalb des Gehäuses so angeordnet ist, dass der Hauptfilter (26) ohne Öffnen des Gehäuses entfernt werden kann.

2. System nach Anspruch 1, **dadurch gekennzeichnet, dass** das System ferner Datenübertragungsmittel (20) zum Senden von durch den Ethylensensor (18) erzeugten Messdaten an einen Server und Aufzeichnungsmittel zum Aufzeichnen der Messdaten umfasst.

3. System nach Anspruch 1 oder 2, **dadurch gekennzeichnet, dass** der Hauptfilter (26) ferner mindestens einen Gewebefilter (262) umfasst, der vorteilhafterweise ein ausgewähltes chemisches Filtermaterial umfasst.

4. System nach einem der Ansprüche 1 bis 3, **dadurch gekennzeichnet, dass** der Hauptfilter (26) des Luftreinigers (10) mindestens einen Kasten (261) einschließt, in der das untergebrachte Filtermaterial einen UV-aktivierten Photokatalysator umfasst, vorteilhafterweise mit Titandioxid beschichtete Glasröhren.

5. System nach einem der Ansprüche 1 bis 4, **dadurch gekennzeichnet, dass** der Hauptfilter (26) des Luftreinigers (10) einen Gewebefilter (262) umfasst, der Aktivkohle enthält und vorteilhafterweise der letzte Filter des Filtersystems in Luftströmungsrichtung ist.

6. System nach einem der Ansprüche 1 bis 5, **dadurch gekennzeichnet, dass** der UV-aktivierte Photokatalysator des Luftreinigers (10) Titandioxid, Kupferoxid oder Zinkoxid ist.

7. System nach einem der Ansprüche 1 bis 6, **dadurch gekennzeichnet, dass** das Filtersystem des Luftreinigers (10) auch Aktivkohle und/oder Nanosilber als Filtermaterial umfasst.

8. System nach einem der Ansprüche 1 bis 7, **dadurch gekennzeichnet, dass** die UV-emittierende Lichtquelle (23) des Luftreinigers (10) eine UVC-emittierende LED mit einer Wellenlänge von vorteilhafterweise 255 bis 275 nm ist.

9. System nach einem der Ansprüche 1 bis 8, **dadurch gekennzeichnet, dass** das Filtersystem des Luftreinigers (10) ferner einen im Strömungskanal angeordneten Vorfilter (24) umfasst, wobei der Vorfilter (24) ein Vliesgewebe umfasst, der ein ausgewähltes chemisches Filtermaterial umfasst.

10. System nach Anspruch 9, **dadurch gekennzeichnet, dass** das Vliesgewebe des Vorfilters (24) des Luftreinigers (10) einen UV-aktivierten Photokatalysator, vorteilhafterweise Titandioxid, umfasst und eine UV-emittierende Lichtquelle (23) auf den Vorfilter (24) gerichtet ist.

11. System nach Anspruch 9 oder 10, **dadurch gekennzeichnet, dass** der Vorfilter (24) des Luftreinigers (10) mittels Gleitschienen (30) so innerhalb des Gehäuses angeordnet ist, dass der Vorfilter (24) ohne Öffnen des Gehäuses entnommen werden kann.

12. System nach einem der Ansprüche 1 bis 11, **dadurch gekennzeichnet, dass** das System ein Softwaremittel einschließt, die so konfiguriert ist, dass sie einen Alarm auslöst, wenn eine vom Ethylensensor (18) gemessene Ethylenkonzentration einen vordefinierten Schwellenwert überschreitet.

13. System nach einem der Ansprüche 1 bis 12, **dadurch gekennzeichnet, dass** das System Softwaremittel zum Auslesen sowohl einer Echtzeit-Ethylenkonzentration als auch eines Parameters einschließt, der den Betrieb des Luftreinigers beschreibt und zu den Messdaten hinzugefügt wird.

14. System nach einem der Ansprüche 1 bis 13, **dadurch gekennzeichnet, dass** der Speicher-/Transportraum eine luftzirkulierende Kältemaschine (54) umfasst, und der Luftreiniger (10) an einer Decke des Lager-/Transportraums in der Nähe der Kältemaschine (54) angeordnet ist, und die Kältemaschine (54) des Speicher- oder Transportraums so konfiguriert ist, dass sie den Luftreiniger (10) mit Leistung versorgt.

15. System nach einem der Ansprüche 1 bis 14, **dadurch gekennzeichnet, dass** das System Softwaremittel zum Regulieren einer Leistung des Ventilators (22) und/oder zum Starten des Ventilators (22) umfasst, wenn die vom Ethylensensor (18) gemessene Ethylenkonzentration einen vorbestimmten Schwellenwert überschreitet.

## Revendications

1. Système de purification d'air dans un espace de stockage/transport fermé qui se compose d'un purificateur d'air (10) comprenant
- un boîtier avec une ouverture d'entrée d'air (11) et une ouverture de sortie d'air (12), dans lequel un canal d'écoulement d'air est situé entre l'ouverture d'entrée d'air (11) et l'ouverture de sortie d'air (12) à l'intérieur du boîtier,
- un ventilateur (22) configuré pour aspirer l'air de l'espace de stockage/transport par le biais de l'ouverture d'entrée d'air (11) dans le canal d'écoulement à l'intérieur du purificateur d'air (10) et pour souffler l'air purifié par le biais de l'ouverture de sortie d'air (12) dans l'espace de stockage/transport,
- un système de filtration disposé dans le canal d'écoulement de telle manière que l'air s'écoule à travers ledit système de filtration, lequel comprend, en tant que matériau de filtration chimique, à la fois un photocatalyseur activé par les rayons UV et un matériau de filtration chimique se composant de permanganate de potassium, et
- une source lumineuse émettant des rayons UV (23) pour activer le photocatalyseur du système de filtration,
- le système de filtration se composant d'un filtre principal (26), qui forme une cassette ayant au moins deux compartiments (261), où le matériau de filtration logé dans le premier compartiment (261) contient du permanganate de potassium et le deuxième compartiment (261) contient un matériau de filtration chimique sélectionné, et où l'air est amené à circuler à travers les compartiments (261),
**caractérisé en ce que**
- le système comprend en outre un capteur d'éthylène qui mesure la concentration d'éthylène dans l'air de l'espace de stockage/transport (18), et
- le filtre principal (26) du purificateur d'air (10) est disposé à l'intérieur du boîtier au moyen de glissières (30) de manière à ce que ledit filtre principal (26) puisse être retiré sans ouvrir le boîtier.

2. Le système conformément à la revendication 1, **caractérisé en ce que** le système comprend en outre un dispositif de transmission de données (20) pour envoyer les données de mesure générées par le capteur d'éthylène (18) à un serveur et un dispositif d'enregistrement pour enregistrer les données de mesure.

3. Le système conformément à la revendication 1 ou 2, **caractérisé en ce que** le filtre principal (26) comprend en outre au moins un filtre en tissu (262), qui se compose avantageusement d'un matériau de filtration chimique sélectionné.

4. Le système conformément à l'une quelconque des revendications 1 à 3, **caractérisé en ce que** le filtre principal (26) du purificateur d'air (10) dispose au moins d'un compartiment (261) dans lequel le matériau de filtration logé comprend un photocatalyseur activé par les rayons UV, avantageusement des tubes de verre enduits de dioxyde de titane.

5. Le système conformément à l'une quelconque des revendications 1 à 4, **caractérisé en ce que** le filtre principal (26) du purificateur d'air (10) dispose d'un filtre en tissu (262) qui se compose de charbon actif et qui est avantageusement le dernier filtre du système de filtration dans le sens de l'écoulement de l'air.

6. Le système conformément à l'une quelconque des revendications 1 à 5, **caractérisé en ce que** le photocatalyseur activé par les rayons UV du purificateur d'air (10) est du dioxyde de titane, de l'oxyde de cuivre ou de l'oxyde de zinc.

7. Le système conformément à l'une quelconque des revendications 1 à 6, **caractérisé en ce que** le système de filtration du purificateur d'air (10) comprend également du charbon actif et/ou du nano-argent comme matériau de filtration.

8. Le système conformément à l'une quelconque des revendications 1 à 7, **caractérisé en ce que** la source lumineuse émettant des rayons UV (23) du purificateur d'air (10) est une DEL émettant des rayons UVC avec une longueur d'onde avantageusement comprise entre 255 et 275 nm.

9. Le système conformément aux revendications 1 à 8, **caractérisé en ce que** le système de filtration du purificateur d'air (10) comprend en outre un préfiltre (24) disposé dans le canal d'écoulement, où ledit préfiltre (24) se compose d'un tissu non tissé qui comprend un matériau de filtration chimique sélectionné.

10. Le système conformément à la revendication 9, **caractérisé en ce que** le tissu non tissé du préfiltre (24) du purificateur d'air (10) dispose d'un photocatalyseur activé par les rayons UV, avantageusement du dioxyde de titane, et qu'une source de lumière émettant des rayons UV (23) est dirigée vers le préfiltre (24).

11. Le système conformément à la revendication 9 ou 10, **caractérisé en ce que** le préfiltre (24) du purificateur d'air (10) est disposé à l'intérieur du boîtier au moyen de glissières (30) de manière à ce que le préfiltre (24) puisse être retiré sans ouvrir le boîtier.

12. Le système conformément à l'une quelconque des revendications 1 à 11, **caractérisé en ce que** le système comprend un dispositif logiciel configuré pour déclencher une alarme si la concentration d'éthylène mesurée par le capteur d'éthylène (18) dépasse un seuil prédéfini.

13. Le système conformément à l'une quelconque des revendications 1 à 12, **caractérisé en ce que** le système comprend un dispositif logiciel pour lire à la fois la concentration d'éthylène en temps réel et un paramètre qui décrit le fonctionnement du purificateur d'air et qui est ajouté aux données de mesure.

14. Le système conformément à l'une quelconque des revendications 1 à 13, **caractérisé en ce que** l'espace de stockage/transport contient une machine frigorifique à circulation d'air (54), que le purificateur d'air (10) est disposé sur le plafond de l'espace de stockage/transport à proximité de la machine frigorifique (54), et que la machine frigorifique (54) de l'espace de stockage ou de transport est configurée de manière à alimenter le purificateur d'air (10) en électricité.

15. Le système conformément à l'une quelconque des revendications 1 à 14, **caractérisé en ce que** le système comprend un dispositif logiciel pour réguler la puissance du ventilateur (22) et/ou pour démarrer le ventilateur (22) lorsque la concentration d'éthylène mesurée par le capteur d'éthylène (18) dépasse le seuil prédéterminé.
